# EUROPEAN PATENT APPLICATION

(11) **EP 4 817 627 A1**
(43) Date of publication of application: **07.10.2026**
(21) Application number: 24896529.5
(22) Date of filing: 26.11.2024
(51) Int. Cl.: A61K 39/395, A61K 47/68, A61K 47/26, A61K 9/19, A61P 35/00

(54) **ANTIBODY FORMULATION**

(30) Priority: 29.11.2023 CN 202311618765; 21.11.2024 CN 202411671978
(71) Applicant: Sichuan Kelun-Biotech Biopharmaceutical Co., Ltd., Chengdu, Sichuan 611138 (CN)
(72) Inventor: WANG, Yiying, Chengdu, Sichuan 611138 (CN); SHAN, Wei, Chengdu, Sichuan 611138 (CN); TANG, Yiting, Chengdu, Sichuan 611138 (CN); LI, Pan, Chengdu, Sichuan 611138 (CN); WANG, Xuemei, Chengdu, Sichuan 611138 (CN); YUAN, Chao, Chengdu, Sichuan 611138 (CN); QUAN, Yong, Chengdu, Sichuan 611138 (CN); OUYANG, Yidan, Chengdu, Sichuan 611138 (CN); TAN, Miao, Chengdu, Sichuan 611138 (CN); TAN, Xiangyang, Chengdu, Sichuan 611138 (CN); GE, Junyou, Chengdu, Sichuan 611138 (CN)
(74) Representative: Meissner Bolte Partnerschaft mbB
(86) International application number: PCT/CN2024/134586
(87) International publication number: WO 2025/113442

(57) **Abstract**

The present disclosure relates to the field of therapeutic antibodies, and specifically, to a pharmaceutical composition comprising an antibody, a buffer solution, and an additional excipient. The pharmaceutical composition can maintain the stability of the antibody after long-term storage and can be directly infused, thereby facilitating clinical use. In the pharmaceutical composition, the antibody component is present at a high concentration, thus featuring convenience of storage and transport and cost-efficiency. In addition, the present disclosure further relates to use of the pharmaceutical composition in preparing a medicament for the prevention and/or treatment and/or adjuvant therapy and/or diagnosis of a tumor.

## Description

### CROSS-REFERENCE TO RELATED APPLICATIONS

The present application claims priority to Chinese Patent Application No. CN2023116187657 filed on Nov. 29, 2023 and Chinese Patent Application No. CN2024116719780 filed on Nov. 21, 2024, which are incorporated herein by reference in their entirety.

### TECHNICAL FIELD

The present disclosure relates to the field of therapeutic antibodies, and particularly, to an anti-EGFR antibody pharmaceutical composition and pharmaceutical use thereof.

### BACKGROUND

Protein pharmaceutical formulations, particularly antibody pharmaceutical formulations, have various modes of administration, such as intravenous, intramuscular, and subcutaneous injections. However, due to the properties of the protein molecules, their liquid formulations often encounter stability problems and impose strict storage conditions. In addition, protein formulations are prone to aggregate or particle formation during the storage, which may affect the biological stability, chemical stability, or physical stability of the formulation.

Recombinant anti-epidermal growth factor receptor (EGFR) human-mouse chimeric monoclonal antibody has a complex higher-ordered structure and poor stability. Existing formulation products of the antibody are prone to the formation of aggregates and protein particles, and an online filtration using a 0.22 µm filter is required prior to the injection. Therefore, for anti-EGFR antibodies, there is a need in the art for antibody formulations with good stability and convenience.

### SUMMARY

The inventors of the present application have surprisingly found, through extensive experiments and repeated explorations, that pharmaceutical antibody compositions of particular components may have excellent stability, contain an active ingredient with a high concentration of protein, and/or do not require dilution or filtration before use. Therefore, the present application relates to a pharmaceutical composition that is particularly beneficial for long-term storage of drugs and clinical use, a method for preparing same, and use thereof. In a first aspect, the present application relates to a pharmaceutical composition, comprising: 0.5-10 g/L of an antibody, 6-12 g/L of a stabilizer, 0.01-0.5 g/L of a surfactant, and a buffer system, wherein the pH of the liquid formulation is 6.0-8.0;
the stabilizer is selected from one or more of sodium chloride, glycine, sucrose, sorbitol, and proline;
the surfactant is selected from polysorbate 20 and polysorbate 80;
the buffer system is selected from a phosphate buffer system, a citrate buffer system, and a histidine buffer system.

In some embodiments, the pharmaceutical composition comprises 1-8 g/L of the antibody, preferably 1-3 g/L or 4-6 g/L of the antibody.

In some embodiments, the pharmaceutical composition comprises 0.5-5 g/L of the antibody, 6-12 g/L of the stabilizer, 0.01-0.5 g/L of the surfactant, and the buffer system, wherein the pH of the liquid formulation is 6.0-8.0;
the stabilizer is selected from one or more of sodium chloride, glycine, sucrose, sorbitol, and proline;
the surfactant is selected from polysorbate 20 and polysorbate 80;
the buffer system is selected from a phosphate buffer system, a citrate buffer system, and a histidine buffer system.

In certain embodiments, the pharmaceutical composition comprises 1.8-2.2 g/L of the antibody, 7.632-9.328 g/L of the stabilizer, 0.05-0.15 g/L of the surfactant, and the buffer system, and the pH of the pharmaceutical composition is 6.4-7.0.

In certain embodiments, the pharmaceutical composition comprises 4-6 g/L of the antibody, 7-10 g/L of the stabilizer, 0.05-0.15 g/L of the surfactant, and the buffer system, and the pH of the pharmaceutical composition is 6.4-7.0.

In certain embodiments, the pharmaceutical composition comprises, on a 1000 mL basis, 1.8-2.2 g/L (e.g., 1.9 g/L, 2.0 g/L, or 2.1 g/L) of the antibody, 7.632-9.328 g/L (0.13 mol/L-0.16 mol/L, e.g., 0.14 mol/L or 0.15 mol/L) of NaCl, 0.05-0.15 g/L (e.g., 0.06 g/L, 0.07 g/L, 0.08 g/L, 0.09 g/L, 0.10 g/L, 0.11 g/L, 0.12 g/L, 0.13 g/L, or 0.14 g/L) of polysorbate 80, 0.369-0.451 g/L (2.7 mmol/L-3.3 mmol/L, e.g., 2.8 mmol/L, 2.9 mmol/L, 3.0 mmol/L, 3.1 mmol/L, or 3.2 mmol/L) of sodium dihydrogen phosphate, and 0.9-1.1 g/L (6.4 mmol/L-7.7 mmol/L, e.g., 6.5 mmol/L, 6.6 mmol/L, 6.7 mmol/L, 6.8 mmol/L, 6.9 mmol/L, 7.0 mmol/L, 7.1 mmol/L, 7.2 mmol/L, 7.3 mmol/L, 7.4 mmol/L, 7.5 mmol/L, or 7.6 mmol/L) of disodium hydrogen phosphate; the pH of the pharmaceutical composition is 6.4-7.0 (e.g., 6.5, 6.6, 6.7, 6.8, or 6.9). In certain embodiments, the pharmaceutical composition comprises, on a 1000 mL basis, 4-6 g/L (e.g., 4.0 g/L, 4.5 g/L, 5.0 g/L, 5.5 g/L, or 6.0 g/L) of the antibody, 7.632-9.328 g/L (0.13 mol/L-0.16 mol/L, e.g., 0.14 mol/L or 0.15 mol/L) of NaCl, 0.05-0.15 g/L (e.g., 0.06 g/L, 0.07 g/L, 0.08 g/L, 0.09 g/L, 0.10 g/L, 0.11 g/L, 0.12 g/L, 0.13 g/L, or 0.14 g/L) of polysorbate 80, 0.369-0.451 g/L (2.7 mmol/L-3.3 mmol/L, e.g., 2.8 mmol/L, 2.9 mmol/L, 3.0 mmol/L, 3.1 mmol/L, or 3.2 mmol/L) of sodium dihydrogen phosphate, and 0.9-1.1 g/L (6.4 mmol/L-7.7 mmol/L, e.g., 6.5 mmol/L, 6.6 mmol/L, 6.7 mmol/L, 6.8 mmol/L, 6.9 mmol/L, 7.0 mmol/L, 7.1 mmol/L, 7.2 mmol/L, 7.3 mmol/L, 7.4 mmol/L, 7.5 mmol/L, or 7.6 mmol/L) of disodium hydrogen phosphate; the pH of the pharmaceutical composition is 6.4-7.0 (e.g., 6.5, 6.6, 6.7, 6.8, or 6.9).

In certain embodiments, the sodium dihydrogen phosphate is a monohydrate.

In certain embodiments, the disodium hydrogen phosphate is an anhydrate.

In certain embodiments, the antibody is an anti-EGFR antibody.

In certain embodiments, the anti-EGFR antibody comprises heavy chain CDRs set forth in SEQ ID NOs: 1-3 and light chain CDRs set forth in SEQ ID NOs: 4-6. In certain embodiments, the anti-EGFR antibody has a heavy chain variable region set forth in SEQ ID NO: 7 and a light chain variable region set forth in SEQ ID NO: 8. In certain embodiments, the anti-EGFR antibody has a heavy chain set forth in SEQ ID NO: 9 and a light chain set forth in SEQ ID NO: 10.

In certain embodiments, the pharmaceutical composition comprises, on a 1000 mL basis, 2.0 g of an anti-EGFR antibody, 8.48 g of sodium chloride, 0.41 g of sodium dihydrogen phosphate (monohydrate), 1.0 g of anhydrous disodium hydrogen phosphate, and 0.1 g of polysorbate 80, wherein the pH of the liquid formulation is 6.7.

In certain embodiments, the pharmaceutical composition comprises, on a 1000 mL basis, 5.0 g of an anti-EGFR antibody, 8.48 g of sodium chloride, 0.41 g of sodium dihydrogen phosphate (monohydrate), 1.0 g of anhydrous disodium hydrogen phosphate, and 0.1 g of polysorbate 80, wherein the pH of the liquid formulation is 6.7.

In a second aspect, the present application relates to another pharmaceutical composition, comprising: 10-100 g/L of an antibody, 50-100 g/L of a stabilizer, 0.05-1.0 g/L of a surfactant, and a buffer system, wherein the pH of the liquid formulation is 5.0-7.5;
the stabilizer is selected from one or more of sodium chloride, glycine, sucrose, sorbitol, and proline;
the surfactant is selected from polysorbate 20 and polysorbate 80;
the buffer system is selected from a phosphate buffer system, a citrate buffer system, and a histidine buffer system.

In certain embodiments, the pharmaceutical composition comprises 20-60 g/L of the antibody, 0.1-0.3 g/L of the surfactant, and 80-90 g/L of the stabilizer, and the pH of the pharmaceutical composition is 5.2-6.2.

In certain embodiments, the pharmaceutical composition comprises, on a 1000 mL basis, 20-60 g/L (e.g., 40 g/L) of the antibody, 10 mmol/L-20 mmol/L (e.g., 11 mmol/L, 12 mmol/L, 13 mmol/L, 14 mmol/L, 15 mmol/L, 16 mmol/L, 17 mmol/L, 18 mmol/L, or 19 mmol/L) of histidine and histidine hydrochloride in total, 0.1-0.3 g/L (e.g., 0.15 g/L, 0.2 g/L, or 0.25 g/L) of polysorbate 80, and 80 g/L-90 g/L (e.g., 81 g/L, 82 g/L, 83 g/L, 84 g/L, 85 g/L, 86 g/L, 87 g/L, 88 g/L, or 89 g/L) of sucrose, and the pH of the pharmaceutical composition is 5.2-6.2 (e.g., 5.3, 5.4, 5.5, 5.6, 5.7, 5.8, 5.9, 6.0, or 6.1).

In certain embodiments, the antibody is an anti-EGFR antibody.

In certain embodiments, the anti-EGFR antibody comprises heavy chain CDRs set forth in SEQ ID NOs: 1-3 and light chain CDRs set forth in SEQ ID NOs: 4-6. In certain embodiments, the anti-EGFR antibody has a heavy chain variable region set forth in SEQ ID NO: 7 and a light chain variable region set forth in SEQ ID NO: 8. In certain embodiments, the anti-EGFR antibody has a heavy chain set forth in SEQ ID NO: 9 and a light chain set forth in SEQ ID NO: 10.

In certain embodiments, the pharmaceutical composition comprises, on a 1000 mL basis, 20 g/L of the anti-EGFR antibody, 10-20 mM of histidine-histidine hydrochloride, 0.2 g/L of polysorbate 80, and 80 g/L of sucrose, wherein the pH of the pharmaceutical composition is 5.5±0.3.

In certain embodiments, the pharmaceutical composition comprises, on a 1000 mL basis, 20 g/L of the anti-EGFR antibody, 20 mM of histidine-histidine hydrochloride, 0.2 g/L of polysorbate 80, and 80 g/L of sucrose, wherein the pH of the pharmaceutical composition is 5.5±0.3.

In certain embodiments, the pharmaceutical composition comprises, on a 1000 mL basis, 20 g/L of the anti-EGFR antibody, 0.67 g/L of histidine, 3.29 g/L of histidine hydrochloride, 0.2 g/L of polysorbate 80, and 80 g/L of sucrose, wherein the pH of the pharmaceutical composition is 5.5±0.3.

In certain embodiments, the pharmaceutical composition comprises, on a 1000 mL basis, 20-60 g/L of the anti-EGFR antibody, 90 g/L of sucrose, 0.1-0.2 g/L of polysorbate 80, and 20 mM histidine-histidine hydrochloride, and the pH of the pharmaceutical composition is 5.5±0.3.

In certain embodiments, the pharmaceutical composition comprises, on a 1000 mL basis, 20 g/L of the anti-EGFR antibody, 90 g/L of sucrose, 0.1 g/L of polysorbate 80, and 20 mM histidine-histidine hydrochloride, and the pH of the pharmaceutical composition is 5.5±0.3. In certain embodiments, the pharmaceutical composition comprises, on a 1000 mL basis, 20-60 g/L of the anti-EGFR antibody, 10 mM of histidine-histidine hydrochloride, 0.2 g/L of polysorbate 80, and 80 g/L of sucrose, wherein the pH of the pharmaceutical composition is 5.5±0.3.

In certain embodiments, the pharmaceutical composition comprises, on a 1000 mL basis, 60 g/L of the anti-EGFR antibody, 10 mM of histidine-histidine hydrochloride, 0.2 g/L of polysorbate 80, and 80 g/L of sucrose, wherein the pH of the pharmaceutical composition is 5.2-5.8.

In certain preferred embodiments, the pharmaceutical composition of the present disclosure can be stable at 5 °C±3 °C and can be stored for at least about 2 weeks, at least about 1 month, at least about 2 months, at least about 3 months, at least about 6 months, at least about 9 months, at least about 1 year, or at least about 2 years. In certain preferred embodiments, the pharmaceutical composition of the present disclosure is stable at about 40 °C and can be stored for at least about 2 weeks, at least about 1 month, at least about 2 months, at least about 3 months, at least about 6 months, at least about 9 months, at least about 1 year, or at least about 2 years. In certain preferred embodiments, the pharmaceutical composition of the present disclosure is stable at room temperature (e.g., about 25 °C) and can be stored for at least about 2 weeks, at least about 1 month, at least about 2 months, at least about 3 months, at least about 6 months, at least about 9 months, at least about 1 year, or at least about 2 years.

In certain embodiments, the strength of the pharmaceutical composition is 150 mg (10 mL)/vial to 2000 mg (10 mL)/vial.

In certain embodiments, the specification of the pharmaceutical composition is selected from the following group: 200 mg (10 mL)/vial, 600 mg (10 mL)/vial, 900 mg (10 mL)/vial, 1200 mg (10 mL)/vial, and 1500 mg (10 mL)/vial.

The pharmaceutical composition of the present disclosure can be prepared by combining the components at predetermined concentrations using methods well known in the art. For example, the antibody can be dialyzed into a solution comprising other components in the liquid formulation and adjusted to a desired concentration. The formulation may be sterilized using a 0.22 µm filter. The prepared pharmaceutical compositions may be packaged for ease of use. The packaging material may be a glass bottle (e.g., a vial), a metal alloy container, a pre-filled syringe, or a pen syringe.

In certain embodiments, the present disclosure provides a method for stabilizing an antibody, wherein the method is implemented by combining 0.5-5 g/L of the antibody, 6-12 g/L of a stabilizer, 0.01-0.5 g/L of a surfactant, and a buffer system, and controlling the pH of the combination at 6.0-8.0.

In certain embodiments, the present disclosure provides a method for stabilizing an antibody, wherein the method is implemented by combining 10-100 g/L of the antibody, 50-100 g/L of a stabilizer, 0.05-1.0 g/L of a surfactant, and a buffer system, and controlling the pH of the combination at 5.0-7.5.

In certain embodiments, the antibody, the surfactant, the stabilizer, the surfactant, and/or the buffer system are as described in any one of the above embodiments.

The pharmaceutical compositions of the present disclosure may be contained in any container suitable for storing a medicament as well as other pharmaceutical compositions. For example, the pharmaceutical composition of the present disclosure may be contained in a sealed and sterilized plastic container, metal alloy container, or glass container with a certain volume, such as an ampoule, a vial, or a syringe. Thus, in certain preferred embodiments, the pharmaceutical composition of the present disclosure is contained in a glass bottle (e.g., an ampoule or vial), a syringe, or a microinfusion device. In certain exemplary embodiments, the pharmaceutical composition of the present disclosure is contained in a glass bottle (e.g., an ampoule or vial).

In certain embodiments, the pharmaceutical composition is a liquid.

In a third aspect, the present disclosure further provides a solid formulation, prepared by lyophilizing the pharmaceutical composition described above. In certain embodiments, the solid formulation is a lyophilized powder for injection.

In a fourth aspect, the present disclosure further provides an article of manufacture, comprising a container containing the pharmaceutical composition of the present disclosure. In certain embodiments, the container is a glass vial, a metal alloy container, or a pre-filled syringe. In certain embodiments, the article of manufacture is a liquid formulation for injection or a solid formulation.

In a fifth aspect, the present application relates to a pharmaceutical formulation, wherein the pharmaceutical formulation is acquired by diluting the pharmaceutical composition according to the second aspect of the present disclosure in a hydrochloric acid-histidine buffer. In certain embodiments, the factor of dilution is 5-50. In certain embodiments, the factor of dilution is 10-30. In certain embodiments, the factor of dilution is 10. In certain embodiments, the pH of the hydrochloric acid-histidine buffer is 5.2-6.2 (e.g., 5.3, 5.4, 5.5, 5.6, 5.7, 5.8, 5.9, 6.0, or 6.1). In certain embodiments, the pharmaceutical formulation is a liquid formulation for injection.

In a sixth aspect, the present application relates to use of the pharmaceutical composition, the pharmaceutical formulation, or the article of manufacture according to any one of the above embodiments in preparing a medicament for:
(a) the prevention and/or treatment of a tumor;
(b) the adjuvant therapy of a tumor;
(c) the diagnosis of a tumor; or
(d) any combination of (a)-(c).

In certain embodiments, the medicament and an additional pharmaceutically active agent are administered separately, jointly, concurrently, or sequentially; optionally, the additional pharmaceutically active agent is a chemotherapeutic agent.

In certain embodiments, the present application provides the pharmaceutical composition, the pharmaceutical formulation, or the article of manufacture according to any one of the above embodiments for use in:
(a) the prevention and/or treatment of a tumor;
(b) the adjuvant therapy of a tumor;
(c) the diagnosis of a tumor; or
(d) any combination of (a)-(c).

In certain embodiments, the present application provides a method for the prevention, treatment, adjuvant therapy, and/or diagnosis of a tumor, comprising: administering to an individual in need a therapeutically effective amount of the pharmaceutical composition, the pharmaceutical formulation, or the article of manufacture according to any one of the above embodiments.

In certain embodiments, the tumor is selected from breast cancer, lung cancer such as non-small cell lung cancer or lung squamous cell carcinoma, liver cancer, gastric cancer, intestinal cancer such as colon cancer or rectal cancer, esophageal cancer, ovarian cancer, cervical cancer, renal cancer, prostate cancer, bladder cancer, pancreatic cancer, Merkel cell carcinoma, bile duct cancer, nasopharyngeal cancer, head and neck squamous cell carcinoma, neuroglioma, melanoma, leukemia, and lymphoma.

In certain embodiments, the tumor is selected from colorectal cancer; preferably, the tumor is selected from RAS wild-type metastatic colorectal cancer.

In certain embodiments, the area under the concentration-time curve (AUC) of the pharmaceutical composition, the pharmaceutical formulation, or the article of manufacture according to any one of the above embodiments in a subject is 5000 h*µg/mL or greater, 6000 h*µg/mL or greater, 7000 h*µg/mL or greater, 8000 h*µg/mL or greater, 9000 h*µg/mL or greater, 10000 h*µg/mL or greater, or 11000 h*µg/mL or greater, and in certain embodiments, no greater than 14000 h*µg/mL.

In certain embodiments, the AUC is AUC₀₋ₜ.

In certain embodiments, the peak concentration (Cₘₐₓ) of the pharmaceutical composition, the pharmaceutical formulation, or the article of manufacture according to any one of the above embodiments in a subject is 50-500 µg/mL, 60-450 µg/mL, 70-400 µg/mL, 80-300 µg/mL, 90-250 µg/mL, or 100-200 µg/mL.

In certain embodiments, the terminal half-life (t_{1/2}) of the pharmaceutical composition, the pharmaceutical formulation, or the article of manufacture according to any one of the above embodiments in a subject is 10 h or greater, 20 h or greater, 30 h or greater, 40 h or greater, or 45 h or greater, and in certain embodiments, no greater than 60 h.

In certain embodiments, the clearance rate (CL) of the pharmaceutical composition, the pharmaceutical formulation, or the article of manufacture according to any one of the above embodiments in a subject is 0.1 L/h or less, 0.09 L/h or less, 0.08 L/h or less, 0.07 L/h or less, 0.06 L/h or less, 0.05 L/h or less, and in certain embodiments, no less than 0.03 L/h.

In certain embodiments, the dose to the subject is, on the basis of body surface area (BSA), 50-500 mg/m², preferably 100-400 mg/m², and more preferably 250 mg/m².

In certain embodiments, 1-5 doses (e.g., 1, 2, 3, 4, or 5 doses), preferably 1 dose, are administered to the subject in one treatment cycle.

In certain embodiments, the route of administration to the subject is oral or intravenous administration, preferably intravenous administration.

In a seventh aspect, the present application relates to use of the pharmaceutical composition, the pharmaceutical formulation, or the article of manufacture according to any one of the above embodiments in preparing a liquid formulation for injection.

### Definitions

In the present disclosure, unless otherwise defined, the scientific and technical terms used herein have the meanings generally understood by those skilled in the art. In addition, the laboratory procedures of cell culture, biochemistry, nucleic acid chemistry, and immunology used herein are the routine procedures widely used in the corresponding fields. Meanwhile, in order to better understand the present disclosure, the definitions and explanations of the related terms are provided below.

As used herein, the term "antibody" is used in the broadest sense and includes a variety of antibody structures, including but not limited to monoclonal antibodies, polyclonal antibodies, multispecific antibodies (e.g., bispecific antibodies), and antibody fragments, so long as they exhibit the desired antigen-binding activity. For example, the "antibody" may be an immunoglobulin molecule that consists of two pairs of polypeptide chains, each pair having one light chain (LC) and one heavy chain (HC). Antibody light chains can be classified into κ (kappa) and λ (lambda) light chains. Heavy chains can be classified into µ, δ, γ, α, or ε, and isotypes of antibodies are defined as IgM, IgD, IgG, IgA, and IgE. In light chains and heavy chains, the variable region and constant region are linked by a "J" region of about 12 or more amino acids, and the heavy chain further comprises a "D" region of about 3 or more amino acids. Each heavy chain consists of a heavy chain variable region (VH) and a heavy chain constant region (CH). The heavy chain constant region consists of 3 domains (CH1, CH2, and CH3). Each light chain consists of a light chain variable region (VL) and a light chain constant region (CL). The light chain constant region consists of one domain CL. The constant domains are not directly involved in the binding of the antibody to the antigen, but exhibit a variety of effector functions, such as mediating the binding of an immunoglobulin to host tissues or factors, including various cells of the immune system (e.g., effector cells) and the first component of the classical complement system (C1q). The VH and VL regions can be further subdivided into hypervariable regions (known as complementarity-determining regions (CDRs)) between which conservative regions known as framework regions (FRs) are distributed. Each VH and VL consists of 3 CDRs and 4 FRs arranged from the amino-terminus to the carboxy-terminus in the following order: FR1, CDR1, FR2, CDR2, FR3, CDR3, FR4. The variable regions (VH and VL) of each heavy chain/light chain pair form an antigen-binding site. The assignment of amino acids in each region or domain follows the definition of Kabat Sequences of Proteins of Immunological Interest (National Institutes of Health, Bethesda, Md. (1987 and 1991)), or Chothia & Lesk (1987) J. Mol. Biol. 196:901-917; Chothia et al. (1989) Nature 342:878-883.

As used herein, unless otherwise clearly defined in the context, when referring to the term "antibody", it includes not only intact antibodies but also antigen-binding fragments of antibodies.

As used herein, the term "complementarity-determining region" or "CDR" refers to the amino acid residues in the variable regions of an antibody that are responsible for antigen binding. The precise boundaries of these amino acid residues may be defined according to various numbering systems known in the art, e.g., the AbM numbering system (Martin ACR, Cheetham JC, Rees AR (1989) Modelling antibody hypervariable loops: A combined algorithm. Proc Natl Acad Sci USA 86:9268-9272) or the IMGT numbering system (Lefranc et al., Dev. Comparat. Immunol. 27:55-77, 2003). For a given antibody, those skilled in the art will readily identify CDRs defined by these numbering systems. Moreover, the correspondence between different numbering systems is well known to those skilled in the art (see, e.g., Lefranc et al., Dev. Comparat. Immunol. 27:55-77, 2003).

In the present disclosure, the CDRs contained in the antibody disclosed herein or an antigen-binding fragment thereof can be determined according to various numbering systems known in the art. In certain embodiments, the CDRs contained in the antibody disclosed herein or an antigen-binding fragment thereof are preferably determined according to the IMGT, Kabat, Chothia, or AbM numbering system.

The term "anti-EGFR antibody" refers to an antibody capable of specifically binding to EGFR. The term "specific binding" refers to that an antibody forms a relatively stable complex in physiological conditions with an antigen. The specific binding may be characterized by an equilibrium dissociation constant (KD) of about 1 × 10⁻⁹ M or less. Methods for determining whether two molecules specifically bind are well known in the art, such as bioluminescent interferometry or surface plasmon resonance techniques (e.g., Biacore).

As used herein, the term "liquid formulation" refers to a combination of at least one active ingredient (e.g., an antibody, which is capable of exerting its biological activity in a subject) and at least one inactive ingredient that, when combined with the active ingredient, is suitable for administration to the subject. Generally, liquid formulations do not contain unacceptable toxic ingredients to a subject to which the formulation would be administered. As used herein, the subject may include a mammal, preferably a human.

As used herein, the expression "stability" of an antibody refers to that the antibody substantially retains its physical and/or chemical stability and/or biological activity after storage. The storage period is generally selected on the basis of a predetermined shelf life of the formulation. Analytical techniques for measuring antibody stability are well known in the art. The stability can be determined at a selected temperature for a selected period (e.g., the antibody stability is determined during at least 28 days of storage at 40 °C, or the antibody stability is determined during at least 12 months of storage at 5 °C±3 °C).

For the physical stability, the antibody in the formulation is considered to retain its physical stability if substantially no signs of aggregation, precipitation, and/or denaturation are found upon visual examination of color and/or clarity, or in UV light scattering or size exclusion chromatography.

The chemical stability can be assessed by detecting and quantifying chemically altered forms of the antibody. Chemical alterations may involve modification in size (e.g., cleavage) that can be evaluated using, for example, size exclusion chromatography, SDS-PAGE, and/or matrix-assisted laser desorption ionization/time-of-flight mass spectrometry (MALDI/TOF MS). Other types of chemical alterations include alterations in charges (e.g., alterations due to deamidation), which can be evaluated, for example, by ion exchange chromatography. For the biological activity, the antibody retains its biological activity in the formulation if it possesses the biological activity of its intended use. For example, if the biological activity of the antibody in the formulation is within about 70-130% (e.g., 80-130%) of the biological activity exhibited upon the preparation of the formulation, it is considered that the biological activity is retained (e.g., as determined by an antigen-binding assay).

As used herein, the term "buffer" refers to a buffered solution that resists the changes in pH by the action of its acid-base conjugate components. Examples of such buffers include acetate, succinate, gluconate, histidine, citrate, glycylglycine, and other organic acid buffers.

The term "histidine buffer" refers to a buffer containing histidine, and the histidine in the buffer may be present alone or in the following forms: e.g., histidine hydrochloride, histidine acetate, histidine phosphate, or histidine sulfate. When histidine is not present alone, the histidine buffer (e.g., a histidine hydrochloride buffer) may be prepared by titrating L-histidine (free base, solid) with the corresponding acid (e.g., hydrochloric acid). When histidine is present alone, a pH regulator may be added as desired in subsequent preparation procedures to give a liquid formulation with an appropriate pH range. In certain preferred embodiments, histidine in the histidine buffer suitable for use in the liquid formulation disclosed herein is present alone or in the form of histidine hydrochloride.

### Beneficial effects

The present disclosure provides a pharmaceutical composition of an antibody (e.g., a recombinant anti-epidermal growth factor receptor (EGFR) human-mouse chimeric monoclonal antibody) that meets various demands, which has one or more of the following advantages: effectively improved stability of the antibody in long-term storage conditions with no protein particles formed; capability to be directly infused and thus convenience in clinical use; and/or the convenience in storage and transportation and cost-efficiency due to the high concentration of the antibody in the pharmaceutical composition.

### DETAILED DESCRIPTION

The present disclosure will now be described with reference to the following examples, which are intended to illustrate the present disclosure, rather than limit the present disclosure. Unless otherwise indicated, the experiments and procedures described in the examples were performed substantially in accordance with conventional methods well known in the art and described in various references. Procedures without specified conditions in the examples are conducted according to conventional conditions or conditions recommended by the manufacturers. Reagents or instruments without specified manufacturers used herein are conventional products that are commercially available. It will be appreciated by those skilled in the art that the examples are illustrative of the present disclosure and are not intended to limit the claimed scope of the present disclosure. All published patents and other references mentioned herein are incorporated herein by reference in their entirety.

### 1. Sequence information

The antibodies involved in the examples (hereinafter referred to as "antibody A") have the sequence information described in Table 1 below.

**Table 1: Description of antibody A sequences**

| **SEQ ID NO.** | **Description** | **Sequence information** |
|---|---|---|
| **1** | **Heavy chain CDR-H1 of antibody A** | **NYGVH** |
| **2** | **Heavy chain CDR-H2 of antibody A** | **VIWSGGNTDYNTPFTS** |
| **3** | **Heavy chain CDR-H3 of antibody A** | **ALTYYDYEFAY** |
| **4** | **Light chain CDR-L1 of antibody A** | **RASQSIGTNIH** |
| **5** | **Light chain CDR-L2 of antibody A** | **YASESIS** |
| **6** | **Light chain CDR-L3 of antibody A** | **QQNNNWPTT** |
| **7** | **Heavy chain variable region of antibody A** | QVQLKQSGPGLVQPSQSLSITCTVSGFSLTNYGVHWVR QSPGKGLEWLGVIWSGGNTDYNTPFTSRLSINKDNSKS QVFFKMNSLQSNDTAIYYCARALTYYDYEFAYWGQGT LVTVSA |
| **8** | **Light chain variable** | DILLTQSPVILSVSPGERVSFSCRASQSIGTNIHWYQQRT NGSPRLLIKYASESISGIPSRFSGSGSGTDFTLSINSVESE |
| | **region of antibody A** | DIADYYCQQNNNWPTTFGAGTKLELK |
| **9** | **Heavy chain of antibody A** | QVQLKQSGPGLVQPSQSLSITCTVSGFSLTNYGVHWVR QSPGKGLEWLGVIWSGGNTDYNTPFTSRLSINKDNSKS QVFFKMNSLQSNDTAIYYCARALTYYDYEFAYWGQGT LVTVSAASTKGPSVFPLAPSSKSTSGGTAALGCLVKDYF PEPVTVSWNSGALTSGVHTFPAVLQSSGLYSLSSVVTVP SSSLGTQTYICNVNHKPSNTKVDKRVEPKSCDKTHTCP PCPAPELLGGPSVFLFPPKPKDTLMISRTPEVTCVVVDV SHEDPEVKFNWYVDGVEVHNAKTKPREEQYNSTYRVV SVLTVLHQDWLNGKEYKCKVSNKALPAPIEKTISKAKG QPREPQVYTLPPSREEMTKNQVSLTCLVKGFYPSDIAVE WESNGQPENNYKTTPPVLDSDGSFFLYSKLTVDKSRWQ QGNVFSCSVMHEALHNHYTQKSLSLSPGK |
| **10** | **Light chain of antibody A** | DILLTQSPVILSVSPGERVSFSCRASQSIGTNIHWYQQRT NGSPRLLIKYASESISGIPSRFSGSGSGTDFTLSINSVESE DIADYYCQQNNNWPTTFGAGTKLELKRTVAAPSVFIFP PSDEQLKSGTASVVCLLNNFYPREAKVQWKVDNALQS GNSQESVTEQDSKDSTYSLSSTLTLSKADYEKHKVYAC EVTHQGLSSPVTKSFNRGEC |

### 2. Source of reagent

Antibody: antibody A used in the examples was a recombinant anti-epidermal growth factor receptor (EGFR) human-mouse chimeric monoclonal antibody with its amino acid sequences shown in Table 1 above. Antibody A was expressed in Chinese hamster ovary (CHO) cells and acquired by serum-free cell culture, isolation, and high-level purification.

Other components: The other components used in the examples were all commercially available products.

### 3. Methodology

The studies involved in the examples adopted the following test methods:
(1) DSF assay
   The sample was diluted with a sample buffer, and the melting curve of the sample was acquired using the Applied Biosystems 7500 qPCR system with the SYPRO^{™} Orange Protein Gel Stain as the dye.
(2) Polysorbate 80 content assay
   The test was performed according to the "Polysorbate 80 residue assay" in General Chapter 3203, Chinese Pharmacopoeia, 2020 Edition.
(3) Insoluble microparticle
   The test was performed according to the "Insoluble microparticle test (light blockage)" in General Chapter 0903, Chinese Pharmacopoeia, 2020 Edition.
(4) Appearance
   The test was conducted by direct visual inspection in a dark room at 1000 lx-1500 lx.
(5) pH value
   The test was performed using a pH meter.
(6) SEC-HPLC
   The G3000SWxl chromatographic column was selected, and a 1% isopropanol-phosphate buffer was selected as the mobile phase for isocratic elution.
(7) IEC-HPLC
   The WCX-10 chromatographic column was selected, and gradient elution was performed on the sample of interest using 10 mM phosphate-100 mM sodium chloride (pH 6.8) as the mobile phase.
(8) Protein content
   100 µL of the sample was placed in a cuvette, the absorbance values of A279-A320 were measured for 3 times to give a mean value, and the protein content was calculated according to the extinction coefficient.

### Example I-1. Stability of formulations with different buffer systems

The stability of formulations I-1-1 and I-1-2 was tested in the conditions of 40±2 °C/75±5% RH (high temperature) and white light (4500 ± 500 lx).
(1) Formulation I-1-1: 2 mg/mL of antibody A, 8.48 g/L of sodium chloride, 1.88 g/L of disodium hydrogen phosphate, and 0.41 g/L of sodium dihydrogen phosphate, pH 7.0.
(2) Formulation I-1-2: 2 mg/mL of antibody A, 7.51 g/L of glycine, 0.1 g/L of polysorbate 80, and 2.10 g/L of citric acid. The stability test results are shown in Table 2:

**Table 2. Stability test results for formulations with different buffers**

| **Test conditions** | **SEC-HPLC main peak (%)** | |
|---|---|---|
| | **Formulation I-1-1** | **Formulation I-1-2** |
| **0 days** | **99.4** | **99.5** |
| **High temperature condition, 10 days** | **98.5** | **96.2** |
| **Irradiation condition, 10 days** | **99.0** | **96.5** |

The results show that formulation I-1-1 using the phosphate buffer system exhibited better stabilities in high temperature condition and irradiation condition than formulation I-1-2 using the citrate buffer system.

Example I-2. Stability test of formulations with different surfactant concentrations Antibody A formulations I-2-A to I-2-C were prepared according to the following compositions:
Formulation I-2-A: on a 1000 mL basis, 2 g of antibody A, 8.48 g of sodium chloride, 1.88 g of disodium hydrogen phosphate heptahydrate, 0.41 g of sodium dihydrogen phosphate monohydrate, and 0.05 g of polysorbate 80;
Formulation I-2-B: on a 1000 mL basis, 2 g of antibody A, 8.48 g of sodium chloride, 1.88 g of disodium hydrogen phosphate heptahydrate, 0.41 g of sodium dihydrogen phosphate monohydrate, and 0.1 g of polysorbate 80;
Formulation I-2-C: on a 1000 mL basis, 2 g of antibody A, 8.48 g of sodium chloride, 1.88 g of disodium hydrogen phosphate heptahydrate, 0.41 g of sodium dihydrogen phosphate monohydrate, and 0.2 g of polysorbate 80.

The above formulations were tested for sub-visible particles. The results are shown in Table 3:

**Table 3. Sub-visible particles test results for formulations**

| **Formulation No.** | **Sub-visible particles ≥ 25 µm (particles/mL)** | |
|---|---|---|
| | **0 days** | **40 °C, agitation at 220 rpm for 3 days** |
| **I-2-A** | **1.0** | **0.4** |
| **I-2-B** | **0.8** | **0.5** |
| **I-2-C** | **0.5** | **0.2** |

In addition, in the condition of agitation at 220 rpm for 3 days at 40 °C, the number of sub-visible particles ≥ 10 µm in the formulations described above was not significantly changed as compared with that on day 0, which met the specification for the quantity of sub-visible particles.

The above results show that the formulations of the present disclosure with different polysorbate 80 concentrations can effectively inhibit the aggregation of antibody A and the production of sub-visible particles and effectively prevent the formation of protein aggregates.

### Example I-3. pH screening test

Antibody A formulations I-3-A to I-3-C were prepared according to the following compositions:
I-3-A: 2 mg/mL antibody A, 8.48 g/L sodium chloride, and 0.1 g of polysorbate 80; a disodium hydrogen phosphate-sodium dihydrogen phosphate buffer system was added to adjust the pH of the formulation to 6.1;
I-3-B: 2 mg/mL antibody A, 8.48 g/L sodium chloride, and 0.1 g of polysorbate 80; a disodium hydrogen phosphate-sodium dihydrogen phosphate buffer system was added to adjust the pH of the formulation to 6.7;
I-3-C: 2 mg/mL antibody A, 8.48 g/L sodium chloride, and 0.1 g of polysorbate 80; a disodium hydrogen phosphate-sodium dihydrogen phosphate buffer system was added to adjust the pH of the formulation to 7.3;

The hydrophobic domain exposure temperatures (*T*ₕ) of the antibody formulations at different pH values were determined by the DSF method. The results are shown in Table 4.

**Table 4. Tₕ of antibody formulations with different pH values**

| **Formulation No.** | ***T*ₕ** |
|---|---|
| **I-3-A** | **71.80** |
| **I-3-B** | **71.66** |
| **I-3-C** | **70.17** |

The results show that within the pH range of 6.1-7.3, there was no significant difference in the hydrophobic domain exposure temperature (T ₕ), suggesting that the antibody has good stability in a formulation within this pH range.

### Example I-4. Stability test of formulations

Antibody A formulation I was prepared according to the following composition: on a 1000 mL basis, 2.0 g of antibody A, 8.48 g of sodium chloride, 0.41 g of sodium dihydrogen phosphate (monohydrate), 1.0 g of anhydrous disodium hydrogen phosphate, and 0.1 g of polysorbate 80, pH 6.7. The stability of antibody A formulation I was tested at 40 °C±2 °C or 5 °C±3 °C in test conditions of various influencing factors. The results are shown in Tables 5-7.

**Table 5. Long-term stability of antibody A formulation I at 5 °C±3 °C**

| **Test item** | | **0 days** | **48 months** |
|---|---|---|---|
| **Appearance** | | **Colorless clear liquid** | **Colorless clear liquid** |
| **SEC-HPLC** | **Main peak (%)** | **98.8** | **98.3** |
| **Protein content** | | **2.0** | **2.0** |

In addition, after 48 months of storage at 5 °C±3 °C, the biological activity data, the quantity of sub-visible particles, and the IEC-HPLC detection data of antibody A formulation I showed no significant difference from those on day 0, and all met the specifications.

As can be seen from the above results, antibody A formulation I of the present disclosure has good stability in long-term conditions, with no significant changes in any indexes after 48 months of storage.

**Table 6. Stability data of antibody A formulation I in high-temperature (40 °C±2 °C) test**

| **Test item** | | **0 days** | **10 days** |
|---|---|---|---|
| **Appearance** | | **Colorless clear liquid** | **Colorless clear liquid** |
| **SEC-HPLC** | **Main peak (%)** | **99.1** | **98.7** |
| **Protein content** | | **2.1** | **2.1** |

In addition, after 10 days of storage at high temperature (40 °C±2 °C), the biological activity data, the quantity of sub-visible particles, and the IEC-HPLC detection data of antibody A formulation I showed no significant difference from those on day 0, and all met the specifications.

As can be seen from the above results, after 10 days of storage at high temperature, all indexes exhibited no significant changes and met the specifications, indicating that antibody A formulation I of the present disclosure has good stability in high-temperature conditions.

**Table 7. Stability data of antibody A formulation I in repeated freeze-thaw (-20 °C/room temperature) conditions**

| **Test item** | | **0 days** | **3 freeze-thaw cycles** |
|---|---|---|---|
| **Appearance** | | **Colorless clear liquid** | **Colorless clear liquid** |
| **SEC-HPLC** | **Main peak (%)** | **99.1** | **99.0** |
| **IEC-HPLC** | **Acidic variants (%)** | **14.1** | **13.8** |
| **Protein content** | | **2.1** | **2.1** |

As can be seen from the data in Table 7, after 3 freeze-thaw cycles at -20 °C/room temperature, all indexes of antibody A formulation I of the present disclosure exhibited no significant changes, indicating that the formulation has good stability in repeated freeze-thaw cycles.

In addition, compared with the dark control group, the SEC-HPLC and IEC-HPLC detection data of antibody A formulation I stored in an irradiation condition for 10 days exhibited no significant difference, indicating that antibody A formulation I of the present disclosure has good stability in an irradiation condition.

Moreover, after antibody A formulation I was stored at an accelerated condition at 25 °C±2 °C for 6 months, the biological activity data, the quantity of sub-visible particles, the SEC-HPLC detection data and the IEC-HPLC detection data of antibody A formulation I showed no significant difference from those on day 0, and all met the specifications.

It can be seen from the above results that antibody A formulation I of the present disclosure has good stability under various influencing factors such as acceleration conditions, long-term conditions, high-temperature conditions, irradiation conditions, and repeated freeze-thaw conditions.

### Example I-5. Formulation stability test

Antibody A formulations I-5-A and I-5-B were prepared according to the following compositions, and their storage stability was tested.

Antibody A formulation I-5-A: on a 1000 mL basis, 2.00 g of antibody A, 8.48 g of sodium chloride, 0.41 g of sodium dihydrogen phosphate (monohydrate), 1.00 g of anhydrous disodium hydrogen phosphate, and 0.10 g of polysorbate 80, pH 6.7,

Antibody A formulation I-5-B: on a 1000 mL basis, 5.00 g of antibody A, 8.48 g of sodium chloride, 0.41 g of sodium dihydrogen phosphate (monohydrate), 1.00 g of anhydrous disodium hydrogen phosphate, and 0.10 g of polysorbate 80, pH 6.7,

The stability test results are detailed in Table 8:

**Table 8. Stability data of formulations at 25 °C±2 °C**

| **Test item** | | **Formulation I-5-A** | | **Formulation I-5-B** | |
|---|---|---|---|---|---|
| | | **0 days** | **3 months** | **0 days** | **3 months** |
| **SEC-HPLC** | **Main peak (%)** | **97.9** | **97.6** | **97.8** | **97.7** |
| | **Aggregates (%)** | **2.1** | **2.0** | **2.2** | **2.0** |

As can be seen from the above results, the main peak and the aggregates contents in the SEC-HPLC chromatograms of the two formulations exhibited no significant changes after 3 months of storage. The low-molecular-weight content by SEC-HPLC chromatography and all the index data by IEC-HPLC and non-reduced and reduced CE-SDS chromatography exhibited no significant changes. These results indicate that the formulations of the present disclosure (e.g., formulations I-5-A and I-5-B) all have good stability.

### Example I-6. Irradiation stability test

Antibody A formulation I-6-A, reference formulation I, and reference formulation II were prepared according to the following compositions, and their stability in irradiation conditions (4500 ± 500 lx, 5 °C± 3 °C) was tested.

Antibody A formulation I-6-A: on a 1000 mL basis, 2.00 g of antibody A, 8.48 g of sodium chloride, 0.41 g of sodium dihydrogen phosphate (monohydrate), 1.00 g of anhydrous disodium hydrogen phosphate, and 0.10 g of polysorbate 80, pH 6.7,

Reference formulation I: on a 1000 mL basis, 5 g of antibody A, 5.84 g of sodium chloride, 7.51 g of glycine, 0.1 g of polysorbate 80, and 2.10 g of citric acid monohydrate, pH 5.3-5.7. Reference formulation II: on a 1000 mL basis, 2 g of antibody A, 8.48 g of sodium chloride, 1.88 g of disodium hydrogen phosphate heptahydrate, and 0.41 g of sodium dihydrogen phosphate monohydrate, pH 7.0-7.4.

**Table 9. Results of irradiation stability test**

| **Formulation No.** | **Test period** | **SEC-HPLC(%)** | |
|---|---|---|---|
| | | **Aggregates** | **Monomer** |
| **Formulation I-5-A** | **0 days** | **2.4** | **97.5** |
| | **Irradiation, 7 days** | **2.4** | **97.5** |
| **Reference formulation I** | **0 days** | **0.3** | **99.5** |
| | **Irradiation, 7 days** | **3.5** | **96.2** |
| **Reference formulation II** | **0 days** | **0.5** | **99.3** |
| | **Irradiation, 7 days** | **2.6** | **97.2** |

As can be seen from the results in Table 9, after 7 days of storage in an irradiation condition, the aggregates and monomer of the formulation of the present disclosure (e.g., formulation I-5-A) exhibited no significant changes. However, both reference formulations exhibited a significant increase in aggregate content and a significant decrease in monomer content. This indicates that the formulation of the present disclosure has significantly better stability in an irradiation condition than the reference formulations.

### Clinical Trial Example

The formulation (hereinafter referred to as "antibody A formulation I") used in the following clinical trial example was: 100 mg (50 mL)/vial, each vial containing 100 mg of antibody A, 424 mg of NaCl, 5 mg of polysorbate 80, 20.5 mg of sodium dihydrogen phosphate (monohydrate), and 50 mg of anhydrous disodium hydrogen phosphate, pH 6.7.

### (1) Efficacy and safety of the formulation of the present disclosure in treating metastatic colorectal cancer

In this study, a total of 18 patients with metastatic colorectal cancer (9 in the low-dose group and 9 in the high-dose group) were enrolled, and the route of administration for antibody A formulation I was intravenous injection. The subjects were observed for 4 weeks after the first dose and administered once every week thereafter. The low dose group was administered at 100 mg/m² for the first dose and 250 mg/m² after the 4 weeks, while the high dose group was administered at 400 mg/m² for the first dose and 400 mg/m² or 250 mg/m² after the 4 weeks. The study results show that in subjects with colorectal cancer, the treatment exhibited acceptable safety at the above doses. 13 subjects were evaluated for efficacy after the treatment, in which 8 in the high dose group achieved PR (partial response), indicating that antibody A formulation I of the present disclosure has good efficacy in the treatment of metastatic colorectal cancer.

### (2) Pharmacokinetics and safety of formulation of the present disclosure

An in vivo pharmacokinetics and safety study was conducted in 80 healthy subjects. 40 subjects in the treatment group received antibody A formulation I at a dose of 250 mg/m², on the basis of the body surface area (BSA). BSA (m²) = [(body weight (kg) × height (cm))/3600]. The treatment was given by a single intravenous dose. By the statistical analysis of the major pharmacokinetic parameter AUC₀₋ₜ and other pharmacokinetic parameters, the results are shown in the table below:

| **Pharmacokinetic parameter** | **Mean±SD** |
|---|---|
| **AUC₀₋ₜ (h*µg/mL)** | **11401.12±2108.45** |
| **Cmax (µg/mL)** | **161.21±22.55** |
| **Tₘₐₓ (h)** | **4.00** |
| **t_{1/2} (h)** | **47.84±11.95** |
| **CL (L/h)** | **0.04±0.01** |
| **V_{d} (L)** | **2.69±0.84** |

| | |
|---|---|
| Note: AUC₀₋ₜ denotes the area under the concentration-time curve from time zero to the last quantifiable concentration. | |

The above results show that the formulation of the present disclosure (e.g., antibody A formulation I) has good pharmacokinetic properties. In terms of safety, no serious adverse events and no deaths were reported. Most treatment-emergent adverse events (TEAEs) occurred a few days after the administration and were mild in severity and mostly relieved without any medical intervention. The proportion of subjects with grade 3 or higher TEAEs was less than 25%. The subjects' physical and vital signs exhibited no treatment-related changes over time, indicating that the formulation of the present disclosure (e.g., antibody A formulation I) has good safety.

(3) Efficacy of formulation of the present disclosure in subjects with RAS wild-type colorectal cancer

52 subjects with RAS wild-type colorectal cancer were enrolled in the study, and the subjects in the treatment group and the control group received antibody A formulation I + mFOLFOX6 and placebo + mFOLFOX6, respectively, as the first-line treatment.

The median progression-free survival (PFS) for subjects with colorectal cancer was 23.89 months for the treatment group and 14.78 months for the control group. The P value of the PFS rate for subjects with colorectal cancer in the treatment group vs. the control group was 0.0389 (P < 0.05), indicating that the differences in PFS of the patients between the two groups were statistically significant. The results show that after the treatment of subjects with RAS wild-type metastatic colorectal cancer, the treatment group (antibody A formulation I in combination with mFOLFOX6) generally exhibited a better efficacy trend as compared with the control group (placebo in combination with mFOLFOX6). Moreover, the safety results show that antibody A formulation I has good safety.

### Example II-1. Stability test of formulations with different compositions

Eight formulations of antibody A, II-1-1 to II-1-8, were prepared. The pH of all formulations was 5.5. The composition of formulation II-1-1 was: 20 g/L of antibody A, 90 g/L of sucrose, and 0.1 g/L of polysorbate 80 in a 10 mM citrate buffer; formulations II-1-2 to II-1-8 were prepared in a 10 mM histidine buffer prepared with histidine and histidine hydrochloride. Formulation II-1-3 contained: 20 g/L antibody A, 90 g/L sucrose, and 0.1 g/L polysorbate 80 in a 10 mM histidine buffer; in formulation II-1-2, the concentration of antibody A was adjusted to 10 g/L on the basis of formulation II-1-3; in formulations II-1-4 to II-1-6, the 90 g/L of sucrose was replaced with 8.48 g/L of sodium chloride, 28.75 g/L of proline, and 50 g/L of sorbitol, respectively, on the basis of formulation II-1-3; in formulation II-1-7, the concentration of polysorbate 80 was adjusted to 0.3 g/L on the basis of formulation II-1-3; in formulation II-1-8, the 0.1 g/L of polysorbate 80 was replaced with 0.1 g/L of polysorbate 20 on the basis of formulation II-1-3.

The stability of the formulation of each group was determined after storage at a high temperature of 40 °C for 28 days. According to the test results, formulation II-1-1 in a citric acid buffer still exhibited significant insoluble particles in appearance, and formulations II-1-2, II-1-3, and II-1-6 to II-1-8 in histidine buffer were still colorless clear liquid in appearance. This indicates that a formulation in a histidine buffer system may have better stability than that in a citric acid buffer system.

In addition, after being stored at a high temperature of 40 °C for 28 days, the appearance of the formulation II-1-2 with an antibody A concentration of 10 g/L and the formulation II-1-3 with an antibody A concentration of 20 g/L was both kept in a colorless clear liquid state, with no significant differences in all indexes of protein content, SEC-HPLC and IEC-HPLC as compared with those on day 0, meeting the specifications. This indicates that all the formulations with different antibody A concentrations have good stability.

The other stability test results are shown in Tables 10 and 11.

**Table 10. Stability test results of formulations with different excipients**

| **Group** | **Sampling time** | **Appearance** | **Protein content (mg/mL)** | **SEC-HPLC** | **IEC** |
|---|---|---|---|---|---|
| | | | | **Main peak (%)** | **Acidic variants (%)** |
| **II-1-3** | **0 days** | **Colorless clear liquid** | **19.7** | **98.8** | **12.3** |
| | **40 °C, 28 days** | **Colorless clear liquid** | **19.4** | **96.7** | **15.7** |
| **II-1-4** | **0 days** | **Colorless clear liquid** | **19.6** | **98.8** | **12.3** |
| | **40 °C, 28 days** | **A large amount of particles like a haze of smoke** | **19.4** | **95.2** | **14.8** |
| **II-1-5** | **0 days** | **Colorless clear liquid** | **20.1** | **98.9** | **12.3** |
| | **40 °C, 28 days** | **A large amount of particles like a haze of smoke** | **19.7** | **96.5** | **16.4** |
| **II-1-6** | **0 days** | **Colorless clear liquid** | **19.8** | **98.9** | **12.3** |
| | **40 °C, 28 days** | **Colorless clear liquid** | **19.4** | **96.7** | **16.4** |

As can be seen from the data in Table 10 above, a large number of particles like a haze of smoke were observed in the sodium chloride group (II-1-4) and the proline group (II-1-5) after storage at 40 °C for 28 days; formulations II-1-3 and II-1-6 exhibited better stability and remained colorless and clear in appearance; in terms of IEC purity, formulation II-1-3 containing sucrose was further superior to formulation II-1-6 containing sorbitol, indicating that sucrose is more beneficial to the stability of the formulation as an excipient.

**Table 11. Stability test results for formulations with different polysorbate concentrations and types**

| **Group** | **Sampling time** | **Appearance** | **Protein content (mg/mL)** | **SEC-HPLC** | **IEC** |
|---|---|---|---|---|---|
| | | | | **Main peak (%)** | **Acidic variants (%)** |
| **II-1-3** | **0 days** | **Colorless clear liquid** | **19.7** | **98.8** | **12.3** |
| | **40 °C, 28 days** | **Colorless clear liquid** | **19.4** | **96.7** | **15.7** |
| **II-1-7** | **0 days** | **Colorless clear liquid** | **19.9** | **98.9** | **12.3** |
| | **40 °C, 28 days** | **Colorless clear liquid** | **19.9** | **96.7** | **15.9** |
| **II-1-8** | **0 days** | **Colorless clear liquid** | **20.1** | **98.9** | **12.3** |
| | **40 °C, 28 days** | **Colorless clear liquid** | **20.3** | **96.6** | **15.8** |

As can be seen from the data in the table above, formulations II-1-3, II-1-7, and II-1-8 with different polysorbate 80 concentrations and polysorbate 20 all have better stability.

Example II-2. Stability test of formulations in long-term and accelerated conditions Formulation II-1-3 in Example II-1 was tested for stability in an accelerated condition (25 °C, 60% RH ± 5% RH) and a long-term condition (5 °C). The test results are shown in Table 12.

**Table 12. Stability test results of formulation II-1-3**

| **Sampling time** | **Protein content (mg/mL)** | **SEC-HPLC Main peak (%)** | **IEC Acidic variants (%)** |
|---|---|---|---|
| **0 days** | **19.7** | **98.8** | **12.3** |
| **25 °C, 3 months** | **19.6** | **98.2** | **12.7** |
| **5 °C, 6 months** | **20.7** | **98.9** | **11.1** |

As can be seen from the data in Table 12, all the indexes of protein content, SEC-HPLC and IEC of injection II-1-3 exhibited no significant changes as compared with those on day 0. In addition, after 6 months of storage at 5 °C, the formulation was still in the form of a colorless clear liquid. The above results indicate that the formulation of the present disclosure has good stability in both accelerated and long-term conditions.

### Example II-3. Stability test of formulations with different antibody concentrations

Three formulations II-3-1 to II-3-3 were prepared according to the compositions shown in Table 13 below, and the stability of each formulation was tested in different storage conditions. The test results are shown in Tables 14 and 15.

**Table 13. Formulations of antibody A injections**

| **Formulatio n No.** | **Buffer system** | **Protein concentration** | **pH** | **Sucrose** | **Polysorbate 80** |
|---|---|---|---|---|---|
| **II-3-1** | **10 mM histidine/histidine hydrochloride buffer** | **20mg/ml** | **5.5** | **80g/L** | **0.2g/L** |
| **II-3-2** | | **40 mg/ml** | | | |
| **II-3-3** | | **60 mg/ml** | | | |

**Table 14. Stability after 6 months of storage at 25 °C**

| **Formulation No.** | **Protein content (mg/mL)** | | **SEC-HPLC main peak (%)** | |
|---|---|---|---|---|
| | **0 days** | **6 months** | **0 days** | **6 months** |
| **II-3-1** | **18.2** | **19.5** | **97.7** | **96.5** |
| **II-3-2** | **37.2** | **39.0** | **97.7** | **96.5** |
| **II-3-3** | **58.7** | **60.5** | **97.6** | **96.1** |

**Table 15. Stability after 12 months of storage at 5 °C**

| **Formulation No.** | **Protein content (mg/mL)** | | **SEC-HPLC main peak (%)** | |
|---|---|---|---|---|
| | **0 days** | **12 months** | **0 days** | **12 months** |
| **II-3-1** | **18.2** | **19.4** | **97.7** | **98.3** |
| **II-3-2** | **37.2** | **39.2** | **97.7** | **98.1** |
| **II-3-3** | **58.7** | **59.4** | **97.6** | **97.8** |

The stability test demonstrated that after the three formulations described above were stored in the long-term condition (5 °C) for 12 months, the high-temperature condition (40 °C) for 30 days, or the accelerated condition (25 °C) for 6 months, the appearance of the samples remained in a colorless clear liquid state, with no significant differences in pH, sub-visible particles, or SEC-HPLC and IEC-HPLC indexes as compared with those on day 0, which met the specifications. This indicates that all the formulations with an antibody concentration of 20-60 mg/mL have good stability.

### Example II-4. Stability test of formulations at different pH

Three formulations II-4-1 to II-4-3 were prepared, and the composition of all the formulations is as follows: 60 mg/mL of antibody A, 80 g/L of sucrose, and 0.2 g/L of polysorbate 80, in a 10 mM histidine/histidine hydrochloride buffer. The pH was 5.5 for II-4-1, 5.2 for II-4-2, and 5.8 for II-4-3. The stability of the formulations was tested in different storage conditions, and the test results are shown in Table 16.

**Table 16. Results of high-temperature stability tests for formulations with different pH values**

| **Group** | **Sampling time** | **Appearance** | **Protein content (mg/mL)** | **SEC-HPLC Main peak (%)** |
|---|---|---|---|---|
| **II-4-1** | **0 days** | **Colorless clear liquid** | **58.6** | **97.6** |
| | **40 °C, 30 days** | **Colorless clear liquid** | **58.7** | **95.0** |
| **II-4-2** | **0 days** | **Colorless clear liquid** | **60.7** | **97.7** |
| | **40 °C, 30 days** | **Colorless clear liquid** | **59.5** | **95.0** |
| **II-4-3** | **0 days** | **Colorless clear liquid** | **60.7** | **97.5** |
| | **40 °C, 30 days** | **Colorless clear liquid** | **59.2** | **94.9** |

In addition, the IEC-HPLC purities of the three formulations exhibited no significant difference from those on day 0, meeting the specification. The above test results demonstrate that all the formulations at pH 5.2-5.8 have good stability.

### Example II-5. Stability test of formulations in different conditions

Antibody A formulation II was prepared according to the following composition: 20 g/L of antibody A, 20 mmol/L of histidine buffer, 0.2 g/L of polysorbate 80, and 80 g/L of sucrose, pH 5.5 ± 0.3. The stability of the formulation was tested in various conditions:

**Table 17. Stability of antibody A formulation II**

| **Group** | **SEC-HPLC** | **IEC-HPLC** | **Protein content (mg/mL)** |
|---|---|---|---|
| | **Monomer(%)** | **Acidic variants (%)** | |
| **0 days** | **99.2** | **9.6** | **19.7** |
| **AG4h** | **99.2** | **9.7** | **19.8** |
| **-70°CFT4X** | **99.2** | **9.7** | **20.5** |
| **5°C1M** | **99.1** | **8.6** | **21.4** |

| | | | |
|---|---|---|---|
| Note: AG4h: agitation at room temperature for 4 h; -70°CFT4X: 4 freeze-thaw cycles at -70 °C/room temperature; 5°C1M: storage at 5 °C for 1 month. | | | |

In addition, after antibody A formulation II was stored at 40 °C for 28 days or under white light irradiation at 4500 lx± 500 lx for 2 weeks, all the test indexes remained stable as compared with those on day 0, with no significant differences found.

As can be seen from the above data, antibody A formulation II of the present disclosure exhibited no significant differences in test indexes in various conditions such as high temperature, agitation, and repeated freeze-thaw cycles as compared with those on day 0 and met the specifications, indicating that the formulation of the present disclosure has good stability against various influencing factors.

The above examples only illustrate several embodiments of the present disclosure for the purpose of specific and detailed description, and should not be construed as limiting the scope of the present disclosure. It should be noted that various modifications and improvements can be made by those of ordinary skill in the art without departing from the spirit of the present disclosure, and such modifications and improvements shall fall within the scope of the present disclosure. Therefore, the scope of the present disclosure is determined by the claims.

## Claims

1. A pharmaceutical composition, comprising: 0.5-10 g/L of an antibody, 6-12 g/L of a stabilizer, 0.01-0.5 g/L of a surfactant, and a buffer system, wherein the pH of the liquid formulation is 6.0-8.0;
the stabilizer is selected from one or more of sodium chloride, glycine, sucrose, sorbitol, and proline;
the surfactant is selected from polysorbate 20 and polysorbate 80;
the buffer system is selected from a phosphate buffer system, a citrate buffer system, and a histidine buffer system.

2. The pharmaceutical composition according to claim 1, comprising: 1-8 g/L of the antibody, preferably 1-3 g/L or 4-6 g/L of the antibody.

3. The pharmaceutical composition according to claim 1 or 2, comprising: 0.5-5 g/L of the antibody, 6-12 g/L of the stabilizer, 0.01-0.5 g/L of the surfactant, and the buffer system, wherein the pH of the pharmaceutical composition is 6.0-8.0.

4. The pharmaceutical composition according to any one of claims 1-3, comprising: 1.8-2.2 g/L of the antibody, 7.632-9.328 g/L of the stabilizer, 0.05-0.15 g/L of the surfactant, and the buffer system, wherein the pH of the pharmaceutical composition is 6.4-7.0; or, comprising: 4-6 g/L of the antibody, 7-10 g/L of the stabilizer, 0.05-0.15 g/L of the surfactant, and the buffer system, wherein the pH of the pharmaceutical composition is 6.4-7.0.

5. The pharmaceutical composition according to any one of claims 1-4, comprising: 1.8-2.2 g/L of the antibody, 7.632-9.328 g/L of NaCl, 0.05-0.15 g/L of polysorbate 80, 0.369-0.451 g/L of sodium dihydrogen phosphate, and 0.9-1.1 g/L of disodium hydrogen phosphate, wherein the pH of the pharmaceutical composition is 6.4-7.0; or, comprising: 4-6 g/L of the antibody, 7.632-9.328 g/L of NaCl, 0.05-0.15 g/L of polysorbate 80, 0.369-0.451 g/L of sodium dihydrogen phosphate, and 0.9-1.1 g/L of disodium hydrogen phosphate, wherein the pH of the pharmaceutical composition is 6.4-7.0.

6. The pharmaceutical composition according to any one of claims 1-5, comprising: 2.0 g of an anti-EGFR antibody, 8.48 g of sodium chloride, 0.41 g of sodium dihydrogen phosphate (monohydrate), 1.0 g of disodium hydrogen phosphate, and 0.1 g of polysorbate 80, wherein the pH of the pharmaceutical composition is 6.7; or, comprising: 5.0 g of an anti-EGFR antibody, 8.48 g of sodium chloride, 0.41 g of sodium dihydrogen phosphate (monohydrate), 1.0 g of anhydrous disodium hydrogen phosphate, and 0.1 g of polysorbate 80, wherein the pH of the liquid formulation is 6.7.

7. A pharmaceutical composition, comprising: 10-100 g/L of an antibody, 50-100 g/L of a stabilizer, 0.05-1.0 g/L of a surfactant, and a buffer system, wherein the pH of the pharmaceutical composition is 5.0-7.5;
the stabilizer is selected from one or more of sodium chloride, glycine, sucrose, sorbitol, and proline;
the surfactant is selected from polysorbate 20 and polysorbate 80;
the buffer system is selected from a phosphate buffer system, a citrate buffer system, and a histidine buffer system.

8. The pharmaceutical composition according to claim 7, comprising: 20-60 g/L of the antibody, 0.1-0.3 g/L of the surfactant, and 80-90 g/L of the stabilizer, wherein the pH of the pharmaceutical composition is 5.2-6.2.

9. The pharmaceutical composition according to claim 7 or 8, comprising, on a 1000 mL basis: 20-60 g/L of the antibody, 10-20 mmol/L of histidine and histidine hydrochloride in total, 0.1-0.3 g/L of polysorbate 80, and 80-90 g/L of sucrose, wherein the pH of the pharmaceutical composition is 5.2-6.2.

10. The pharmaceutical composition according to claim 9, comprising, on a 1000 mL basis: 20 g/L of the anti-EGFR antibody, 0.67 g/L of histidine, 3.29 g/L of histidine hydrochloride, 0.2 g/L of polysorbate 80, and 80 g/L of sucrose, wherein the pH of the pharmaceutical composition is 5.5±0.3.

11. The pharmaceutical composition according to any one of claims 1-10, wherein the antibody is an anti-EGFR antibody.

12. The pharmaceutical composition according to claim 11, wherein the anti-EGFR antibody comprises heavy chain CDRs set forth in SEQ ID NOs: 1-3 and light chain CDRs set forth in SEQ ID NOs: 4-6.

13. The pharmaceutical composition according to claim 12, wherein the anti-EGFR antibody has a heavy chain variable region set forth in SEQ ID NO: 7 and a light chain variable region set forth in SEQ ID NO: 8.

14. The pharmaceutical composition according to claim 13, wherein the anti-EGFR antibody has a heavy chain set forth in SEQ ID NO: 9 and a light chain set forth in SEQ ID NO: 10.

15. A solid formulation, prepared by lyophilizing the pharmaceutical composition according to any one of claims 1-14.

16. An article of manufacture, comprising a container containing the pharmaceutical composition according to any one of claims 1-14 or the solid formulation according to claim 15.

17. The article of manufacture according to claim 16, wherein the container is a glass bottle, a metal alloy container, or a pre-filled syringe.

18. A pharmaceutical formulation, wherein the formulation is acquired by diluting the pharmaceutical composition according to any one of claims 7-14 in a buffer.

19. The pharmaceutical formulation according to claim 18, wherein the formulation is acquired by diluting the pharmaceutical composition according to any one of claims 7-14 in a histidine hydrochloride-histidine buffer.

20. The pharmaceutical formulation according to claim 19, wherein the liquid formulation is acquired by diluting the pharmaceutical composition according to any one of claims 7-14 5- to 50-fold in the histidine hydrochloride-histidine buffer.

21. The pharmaceutical formulation according to claim 20, wherein the liquid formulation is acquired by diluting the pharmaceutical composition according to any one of claims 7-14 10-fold in the histidine hydrochloride-histidine buffer.

22. The pharmaceutical formulation according to any one of claims 19-21, wherein the pH of the histidine hydrochloride-histidine buffer is 5.2-6.2.

23. A method for stabilizing an antibody, comprising combining the antibody, the stabilizer, the surfactant, and/or the buffer system according to any one of claims 1-14, and controlling the pH of the combination in the range according to any one of claims 1-10.

24. Use of the pharmaceutical composition according to any one of claims 1-14, the solid formulation according to claim 15, the article of manufacture according to claim 16 or 17, or the pharmaceutical formulation according to any one of claims 18-22 in preparing a medicament for:
(a) the prevention and/or treatment of a tumor;
(b) the adjuvant therapy of a tumor;
(c) the diagnosis of a tumor; or
(d) any combination of (a)-(c).

25. The use according to claim 24, wherein the medicament and an additional pharmaceutically active agent are administered separately, jointly, concurrently, or sequentially; optionally, the additional pharmaceutically active agent is a chemotherapeutic agent.

26. The use according to claim 24 or 25, wherein the tumor is selected from breast cancer, lung cancer such as non-small cell lung cancer or lung squamous cell carcinoma, liver cancer, gastric cancer, intestinal cancer such as colon cancer or rectal cancer, esophageal cancer, ovarian cancer, cervical cancer, renal cancer, prostate cancer, bladder cancer, pancreatic cancer, Merkel cell carcinoma, bile duct cancer, nasopharyngeal cancer, head and neck squamous cell carcinoma, neuroglioma, melanoma, leukemia, and lymphoma.

27. Use of the pharmaceutical composition according to any one of claims 1-14, the solid formulation according to claim 15, or the pharmaceutical formulation according to any one of claims 18-22 in preparing a liquid formulation for injection.
